(19) Europäisches Patentamt / European Patent Office / Office européen des brevets

(11) **EP 2 767 240 B1**

## (12) EUROPEAN PATENT SPECIFICATION

(45) Date of publication and mention
of the grant of the patent:
**04.05.2022 Bulletin 2022/18**

(21) Application number: **11874008.3**

(22) Date of filing: **11.10.2011**

(51) International Patent Classification (IPC):
**A61B 17/00** (2006.01)      **A61B 90/00** (2016.01)
**G01C 21/20** (2006.01)      **G01C 25/00** (2006.01)
**G06F 17/40** (2006.01)

(52) Cooperative Patent Classification (CPC):
**G16Z 99/00; A61B 34/20; G16H 20/40;
G16H 40/40;** A61B 2017/00725; G06F 17/40

(86) International application number:
**PCT/CN2011/080636**

(87) International publication number:
**WO 2013/053103 (18.04.2013 Gazette 2013/16)**

(54) **DETERMINATION METHOD FOR DIRECTIONAL CALIBRATION PARAMETERS AND ACTION DIRECTION OF SURGICAL INSTRUMENT**

BESTIMMUNGSVERFAHREN FÜR GERICHTETE KALIBRATIONSPARAMETER UND AKTIONSRICHTUNG CHIRURGISCHER INSTRUMENTE

PROCÉDÉ DE DÉTERMINATION DES PARAMÈTRES D'ÉTALONNAGE DIRECTIONNEL ET DE LA DIRECTION D'ACTIONNEMENT D'UN INSTRUMENT CHIRURGICAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**20.08.2014 Bulletin 2014/34**

(73) Proprietor: **Ji, Ying**
**Zhejiang (CN)**

(72) Inventors:
• **Ji, Ying**
**Nanjing City, Jiangsu 211199 (CN)**
• **Ji, Jiansong**
**Lishui City, Zhejiang Province (CN)**

(74) Representative: **Barker Brettell LLP**
**100 Hagley Road**
**Edgbaston**
**Birmingham B16 8QQ (GB)**

(56) References cited:
CN-A- 101 019 771      CN-A- 101 099 673
CN-A- 101 879 092      US-A1- 2005 288 575
US-A1- 2007 083 133    US-A1- 2007 173 790
US-A1- 2008 039 868    US-A1- 2009 247 861
US-B2- 7 043 961

**Description**

Technical Field

The present invention relates to methods in positional tracking systems to determine the directional calibration parameters and the action direction of a tracked object.

BACKGROUND OF THE INVENTION

In mini-invasive surgery, there is one kind of procedures, percutaneous puncture. Examples are biopsy, punctures for ablation treatment of liver cancer and lung cancer. In such procedure, a needle-like surgical instrument is punctured into a patient's body for its tip to reach the region of interest for diagnosis or treatment.

In orthopedics surgery, nail or needle-like instrument is often implanted/inserted into a patient's body for cutting/treating some area. In radiotherapy, the direction of particle implantation needs to be determined. In neurosurgery, drilling into a patient's cranium needs an accurate direction. In the medical field, the direction of some surgical instruments may need to be controlled accurately during operation. The precise determination of the action direction of the surgical instrument can play an important role in the surgical outcome.

Applying a surgical navigation system is a good way to achieve a better accuracy for certain surgical procedures. The surgical navigation system includes position tracking apparatus, surgical navigation software, etc. The surgical navigation system can track a surgical instrument's movement in the three dimensional space and display an image of the surgical instrument's movement with respect to the anatomic image of the patient on a monitor. These images provide a visual tool to guide the procedure for surgeons.

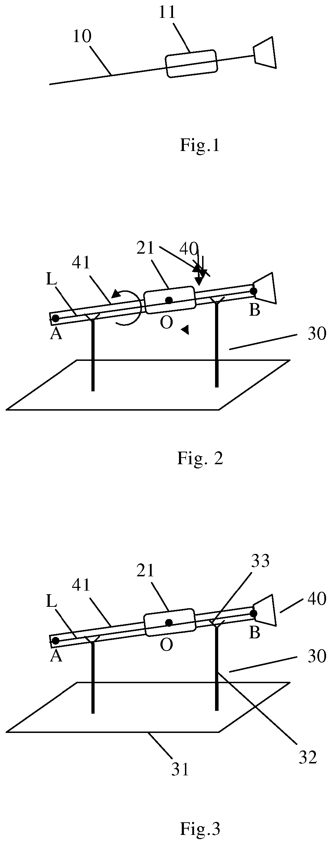

As shown in Figure 1, in order to track the trajectory of the surgical needle 10, a tracking marker 11 is mounted on the surgical needle 10, so that the position and direction of the tracking marker 11 can be tracked via the positional tracking apparatus, and thereby the trajectory of the surgical needle 10 can be determined. Now there are two kinds of positional tracking apparatus: infrared tracking apparatus and electromagnetic tracking apparatus. When using an infrared tracking apparatus, one or more tracking markers may be mounted on the surgical instrument. The markers can emit infrared light (active mode), or reflect infrared light (passive mode). Through the computation over the transmission of the emitted/reflected infrared light, the infrared tracking apparatus can deduce the tracking markers' spatial positions and directions of movement. When using an electromagnetic tracking apparatus, one or more tracking markers, for example, comprising sensing coils, can be mounted on the surgical instrument to be tracked. Through the computation over the induced voltage on the sensing coil, the electromagnetic tracking apparatus can deduce the tracking markers' positions and directions of movement. By following the tracking markers' movement, the positional tracking apparatus can provide information of the positions and directions of movement of the tracked object in a real-time manner.

In practice, an object often has its distinctive geometric shape, which causes the tracking marker's spatial position and direction to represent only for itself, but not so much for the tracked object. How to accurately indicate the action direction of the tracked surgical instrument via the direction of the tracking marker involves a calibration issue.

One approach for this issue is to initially place the tracked surgical instrument at a known direction, measure the direction parameters of the tracking marker from the positional tracking apparatus, compute the deviation between the known direction of the tracked surgical instrument and the direction of the tracking marker . The deviation is regarded as the directional calibration parameters. When the surgical instrument moves in any direction, based on the direction of the tracking marker and by applying the previously determined directional calibration parameters to compensate, the action direction of the tracked surgical instrument can be obtained. However, the surgical instrument may need to be placed initially in a known direction and the precise placement at a known direction may be difficult to perform. The known direction in space with respect to the positional tracking apparatus may be particularly difficult to determine precisely in the three-dimensional space.

Another approach is to use two points on the object to determine the directional calibration parameters. Although it may be easier to determine the calibration parameters for one point on an object, it may be more difficult to determine the calibration parameters for another point because of the object's geometry.

In the application of tracking a surgical needle, for example, the calibration parameters of the surgical needle's tip may be more easily determined because the tip itself can be regarded as a point. In contrast, however, the calibration parameters for a point on the needle's tail may be more difficult to measure because the tail is often not a geometric point. When the distance between the position of the tracking marker and the position of the needle tip (if it is not convenient for the tracking marker to be mounted on the tip), if the computed directional calibration parameters are not sufficiently accurate, significant positional errors may be present along the needle's elongated direction. As such, in many cases the tracking marker is mounted directly on the tip of a hollow needle, so that the deviation is small between the needle and the tracking marker, allowing for a better operational accuracy.

US2009/247861A1 describes a calibration method for an axially determinate medical instrument, wherein the instrument, which is situated within the localization range of a medical tracking system, is positioned such that it can be rotated about its spatially fixed or spatially determinate axis, the instrument is rotated about the axis, wherein a reference which is situated on the instrument such that is spatially fixed relative to the instrument comes to rest at at least two positions, the at least two positions are spatially determined with the aid of the tracking system, the axis of the instrument is spatially determined from the determined position, and wherein the axis thus determined is assigned to the tracked instrument.

**Summary**

To solve the above-mentioned drawbacks, methods and apparatus for accurately determining directional calibration parameters and directions of movement of a tracked object are provided.

A method for determining the directional calibration parameters of a tracked surgical instrument is provided in accordance with claim 1. Some optional or preferred features are set out in dependent claims

A method of determining the action direction of a tracked surgical instrument is provided in accordance with claim 3. Some optional or preferred features are set out in dependent claims.

Advantages

Compared with existing methods which need to place the surgical instrument in a known direction, the present invention's method of determining the directional calibration parameters does not have such a requirement. By applying the positional tracking apparatus, the directional calibration parameters can be computed via the rotation status parameters of the tracking marker mounted on the measured object (the surgical instrument or the replacement). The operation is easy.

Furthermore, the method of determining the directional calibration parameters does not require computing the calibration parameters for two-point set on the straight line representing the direction of the measured object, and has nothing to do with the positions of the two points.

The method for determining the direction for the surgical instrument is: obtaining current rotating status parameters, using prior known directional calibration parameters to deduce the direction. The method does not require determining a set of two points on the linear direction for tracked surgical instrument. The linear direction represents the action direction of the surgical instrument. The action direction information for some surgical instruments is important for doctors to achieve a precise procedure, a better surgical outcome.

The present invention is applicable to any surgical instrument that needs to determine some linear direction on whole or part of the instrument, such as surgical needles, surgical drills, and implant screws. One more example is a surgical clamp with flexible endoscope. Though its hose part has no linear direction, head for removal and treatment, has action direction. Its action direction can be determined by the present invention's methods. Further, the present invention is also applicable to the case that the linear direction is not from the surgical instrument, but is emitted from the surgical instrument. For example, radiation, lasers, or particles emitted from the surgical instrument have linear direction, which can also be determined by the present invention.

The present invention is applicable to any case that needs to determine the linear direction of an object with positional tracking apparatus. The action direction may be a linear direction on the whole tracked object, or on the part of the tracked object, or may be located outside.

The present invention's tool used specially for determining the directional calibration parameters plays a role to support, clamp and fix the measured object. The tool can assistant the measured object to rotate along the set line as axis while keeping the direction of line unchanged. The tool can ensure fast and accurate measurement of the directional calibration parameters.

BRIEF DESCRIPTION OF THE DRAWINGS

Figure 1 is a schematic view of the tracking marker mounted on the surgical needle;

Figure 2 is a positional diagram for points A, B on the measured object and point C on the tracking marker mounted on the measured object;

Figure 3 is a block diagram showing an embodiment of the calibration tool;

Figure 5 is a flow chart of an embodiment of the method to determine the directional calibration parameters;

Figure 7 is a flow chart of an embodiment of the method to determine the action direction;

DETAILED DESCRIPTION

The following description includes three sections: 1. determining the directional calibration parameters for a surgical instrument; 2. determining the direction for a surgical instrument ; and 3. a tool used in determining the directional calibration parameters.

1. Determining the directional calibration parameters for a surgical instrument

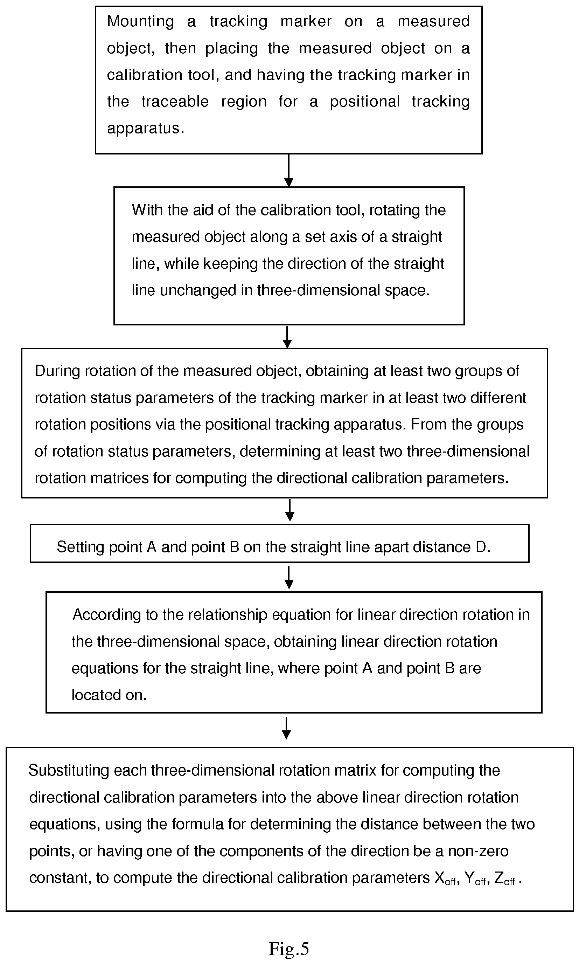

Figure 5 shows a flow chart of applying an embodiment of a method for determining the directional calibration parameters for the tracked surgical instrument. As shown in Figure 2 and Figure 5, the method for determining the directional calibration parameters comprises the steps of:

a) disposing a tracking marker 21 over a measured object 40, then placing the measured object 40 on a calibration tool 30, and having the tracking marker 21 in the traceable region for a positional tracking apparatus.

b) with the aid of the calibration tool 30, rotating the measured object 40 along a set axis of a straight line L, while keeping the direction of the straight line L unchanged in the three-dimensional space.
(The straight line L set for the measured object 40 may be located on the whole measured object 40, or on the part of the measured object, may also be located outside the measured object 40).,

c) during rotation of the measured object 40, obtaining at least two groups of rotation status parameters of the tracking marker 21 in at least two different rotation positions via the positional tracking apparatus. From the groups of rotation status parameters, determining at least two three-dimensional rotation matrices for computing directional calibration parameters.

d) setting point A and point B on the set straight line with a distance D apart (D is larger than 0); setting three-dimensional coordinates A $(X_A, Y_A, Z_A)$, B $(X_B, Y_B, Z_B)$ and C $(X_C, Y_C, Z_C)$ respectively;

e) setting $\delta x = X_A - X_B$, $\delta y = Y_A - Y_B$, $\delta z = Z_A - Z_B$, wherein $\delta x$, $\delta y$, $\delta z$ represent X, Y, Z components of the linear direction in three-dimensional coordinate system.

When the tracking marker 21 is at "zero" direction, its three rotation angles are all zero, its rotation matrix will be unit matrix, x, y, and z components of direction of line can be expressed as $X_{off} = \delta x^{(0)}$, $Y_{off} = \delta y^{(0)}$, $Z_{off} = \delta z^{(0)}$.
According to the formula of linear direction rotation relationship, equation 10)-12) are obtained:

$$\delta x = X_{off} \times M_p(1,1) + Y_{off} \times M_p(2,1) + Z_{off} \times M_p(3,1) \qquad 10) \; ;$$

$$\delta y = X_{off} \times M_p(1,2) + Y_{off} \times M_p(2,2) + Z_{off} \times M_p(3,2) \qquad 11) \; ;$$

$$\delta z = X_{off} \times M_p(1,3) + Y_{off} \times M_p(2,3) + Z_{off} \times M_p(3,3) \qquad 12) \; ;$$

Wherein: $X_{off}$, $Y_{off}$. $Z_{off}$ are x,y,z components of directional calibration parameters for the set straight line for the measured object.
The matrix $M_p$

$$\begin{bmatrix} M_p(1,1) & M_p(1,2) & M_p(1,3) \\ M_p(2,1) & M_p(2,2) & M_p(2,3) \\ M_p(3,1) & M_p(3,2) & M_p(3,3) \end{bmatrix}$$

is the Pth matrix of three-dimensional rotation for computing directional calibration parameters determined from step C), p=1, 2, ..., n, where n is a positive integer greater than 1.

f) substituting each three-dimensional rotation matrix for computing directional calibration parameters determined from step C) into equation 10) -12), using the formula for determining the distance between the two points, or having one of the components of the direction be a non-zero constant, to compute the directional calibration parameters $X_{off}$, $Y_{off}$, $Z_{off}$

Some implementations to use the formula for determining the distance between the two points in step f) are as follows:

The formula of distance D between point A and point B is:

$$D = \sqrt{\delta x^2 + \delta y^2 + \delta z^2} \; ;$$

wherein: D is a known non-zero number constant. Substituting each three-dimensional rotation matrix for computing directional calibration parameters determined from step C) into equations 10)-12), plus the equation of D, constitutes simultaneous equations. Solving the combined simultaneous equations gives $X_{off}$, $Y_{off}$, $Z_{off}$.

Some implementations to have one of the components of the direction be a non-zero constant in step f) are as follows:

Among $\delta x$, $\delta y$, $\delta z$, setting a non-zero component as known non-zero constant. Substituting each three-dimensional rotation matrix for computing directional calibration parameters determined from step C) into equations 10)-12), constitutes non-homogeneous linear equations. Solving the non-homogeneous linear equations gives $X_{off}$, $Y_{off}$, $Z_{off}$.

It is noted for the formula of linear direction rotation relationship that when the tracking marker 21 is at "zero" direction, its three rotation angles are all zero, its rotation matrix will be unit matrix. x, y, and z components of direction of line L can be expressed as $X_{off} = \delta x^{(0)}$, $Y_{off} = \delta y^{(0)}$, $Z_{off} = \delta z^{(0)}$. When the tracking marker 21 is at any direction, its rotation matrix will not be unit matrix. The direction of line L will be its "zero" direction $X_{off}$, $Y_{off}$, $Z_{off}$ (x, y, z components) multiplied by the rotation matrix, which is just what the equation 10)-12) describe.

Some implementations to have one of the components of the direction be a non-zero constant in step f) are based on the following principle:

When disposing the tracking marker 21 mounted on the measured object 40 in the traceable region for a positional tracking apparatus, the direction of the set line L for the measured object 40 can be in anywhere. It is publicly known that at least one of the linear direction x, y, z components is not zero (in other words, the line is not perpendicular to at least one of x, y, z axes). Among $\delta x$, $\delta y$, $\delta z$, setting a non-zero component as known non-zero constant is reasonable.

It may be noted that $X_{off}$, $Y_{off}$, $Z_{off}$ can be determined in the invention's method such that they are not dependent on the positions of the two points A, B on the line L. Even if the measured object changes its position during rotation, the determination of the directional calibration parameters will not be affected, as long as the direction of set line for the measured object remains unchanged.

Below is the description of using two three-dimensional rotation matrices to compute the directional calibration parameters, by having one of the components of the direction be a non-zero constant, or by using the formula for determining the distance between two points.

Suppose that $M_1$ and $M_2$ are two determined three-dimensional rotation matrix for determining directional calibration and are expressed as:

$$\begin{bmatrix} M_1(1,1) & M_1(1,2) & M_1(1,3) \\ M_1(2,1) & M_1(2,2) & M_1(2,3) \\ M_1(3,1) & M_1(3,2) & M_1(3,3) \end{bmatrix}$$

and

$$\begin{bmatrix} M_2(1,1) & M_2(1,2) & M_2(1,3) \\ M_2(2,1) & M_2(2,2) & M_2(2,3) \\ M_2(3,1) & M_2(3,2) & M_2(3,3) \end{bmatrix}$$

Then from equations 10)-12), below equations 1-1) - 1-6) are obtained:

$$\delta x = X_{off} \times M_1(1,1) + Y_{off} \times M_1(2,1) + Z_{off} \times M_1(3,1) \qquad 1\text{-}1\,\rangle\; ;$$

$$\delta y = X_{off} \times M_1(1,2) + Y_{off} \times M_1(2,2) + Z_{off} \times M_1(3,2) \qquad 1\text{-}2\,\rangle\; ;$$

$$\delta z = X_{off} \times M_1(1,3) + Y_{off} \times M_1(2,3) + Z_{off} \times M_1(3,3) \qquad 1\text{-}3\,\rangle\; ;$$

$$\delta x = X_{off} \times M_2(1,1) + Y_{off} \times M_2(2,1) + Z_{off} \times M_2(3,1) \qquad 1\text{-}4\,\rangle\; ;$$

$$\delta y = X_{off} \times M_2(1,2) + Y_{off} \times M_2(2,2) + Z_{off} \times M_2(3,2) \qquad 1\text{-}5\,\rangle\; ;$$

$$\delta z = X_{off} \times M_2(1,3) + Y_{off} \times M_2(2,3) + Z_{off} \times M_2(3,3) \qquad 1\text{-}6\,\rangle\; ;$$

In some implementations, by using the formula for determining the distance between two points:

$$D = \sqrt{\delta x^2 + \delta y^2 + \delta z^2}$$

$X_{off}$, $Y_{off}$, $Z_{off}$ can be solved via simultaneous equations 1-1) - 1-6) and the equation of D. Wherein D is a known non-zero constant.

Some implementations to have one of the components of the direction be a non-zero constant are as follows: Among $\delta x$, $\delta y$, $\delta z$, set a non-zero component as known non-zero constant. For example, the measured object is seated in a direction that is not perpendicular to X axis. Then $\delta x$ is not zero, $\delta x$ = constant. Then equations 1-1)-1-6) become equations 1-7) -1-12) as following:

$$\mathrm{const} = X_{off} \times M_1(1,1) + Y_{off} \times M_1(2,1) + Z_{off} \times M_1(3,1) + 0 + 0 \qquad 1\text{-}7\,\rangle\; ;$$

$$0 \quad = X_{off} \times M_1(1,2) + Y_{off} \times M_1(2,2) + Z_{off} \times M_1(3,2) - \delta y + 0 \qquad 1\text{-}8\,\rangle\; ;$$

$$0 \quad = X_{off} \times M_1(1,3) + Y_{off} \times M_1(2,3) + Z_{off} \times M_1(3,3) + 0 - \delta z \qquad 1\text{-}9\,\rangle\; ;$$

$$\mathrm{const} = X_{off} \times M_2(1,1) + Y_{off} \times M_2(2,1) + Z_{off} \times M_2(3,1) + 0 + 0 \qquad 1\text{-}10\,\rangle\; ;$$

$$0 \quad = X_{off} \times M_2(1,2) + Y_{off} \times M_2(2,2) + Z_{off} \times M_2(3,2) - \delta y + 0 \qquad 1\text{-}11\,\rangle\; ;$$

$$0 \quad = X_{off} \times M_2(1,3) + Y_{off} \times M_2(2,3) + Z_{off} \times M_2(3,3) + 0 - \delta z \qquad 1\text{-}12\,\rangle\; ;$$

Equations of 1-7)-1-12) compose a set of non-homogeneous linear equations regarding unknown numbers of $X_{off}$, $Y_{off}$, $Z_{off}$, $\delta x$, $\delta y$, $\delta z$. Solving the equation set will give $X_{off}$, $Y_{off}$, $Z_{off}$.

If using more than two three-dimensional rotation matrices, such $M_3$, $M_4$ ..., to solve $X_{off}$, $Y_{off}$, $Z_{off}$, the number of equations may be more than the number of unknowns. Solving further equations by other methods (e.g., using an iterative method) will obtain a more accurate solution for $X_{off}$, $Y_{off}$, $Z_{off}$. This is a well-known technique, and is not detailed here.

In the method of determining the directional calibration parameters, the measured object may be the surgical instrument or the replacement for the surgical instrument for determining the directional calibration parameters.

When the measured object is the surgical instrument itself, the method just measures the directional calibration parameters for the surgical instrument itself. During tracking and determining the direction of the surgical instrument, the tracking marker is mounted on the surgical instrument, in a way that the relative direction or angle in the three-dimensional space between the direction of the straight line for the surgical instrument and the tracking marker is substantially the same as the relative direction or angle in the three-dimensional space between the direction of the straight line for the surgical instrument and the tracking marker during the determination of the directional calibration parameters. There is no relative rotation, but translation in three-dimensional space.

When the surgical instrument is not convenient to dispose on the calibration tool for measuring the directional calibration parameters, a replacement may be used. During tracking and determining the direction of the surgical instrument, the tracking marker is mounted on the surgical instrument, in a way that the relative direction or angle in the three-dimensional space between the direction of the straight line for the surgical instrument and the tracking marker is substantially the same as the relative direction or angel in the three-dimensional space between the direction of the straight line for the replacement and the tracking marker during the determination of the directional calibration parameters. There is no relative rotation, but translation in three-dimensional space.

In some implementations, the straight line L set for the measured object 40 may be located on the measured object 40, may also be located outside the measured object 40, as long as the measured object 40 is rotatable along the set straight line L as its rotation axis. As shown in Figure 2, the measured object is a cylindrical needle. The cylindrical part 41 's axis L is the set line for the measured object. Point A and B are located at the axis L. The tracking marker may be mounted anywhere on the measured object..

The mounting of the tracking marker on the measured object is fixed so that there is no relative direction change between the tracking marker and the measured object in the three-dimensional space when the rotation status parameters of the tracking marker are acquired.

The tracking marker fixed on the tracked object may be detachable. When reattaching the tracking marker, it is ensured that there is no relative direction change between the tracking marker and the measured object in the three-dimensional space.

The three-dimensional rotation matrix can be obtained according to the rotation status parameters of the tracking marker 21 using a positional tracking apparatus. In some implementations, rotation angles of the tracking marker 21 are used to obtain the three-dimensional rotation matrix for computing the directional calibration parameters.

In some implementations, the tracking marker is disposed in front of the positional tracking apparatus, so that the tracking marker is in the traceable region and can be tracked to obtain its three-dimensional rotation status parameters, and therefore its three-dimensional rotation matrix.

In some implementations, the rotation of the measured object 40 may be manually or automatically implemented.

In some implementations, the calibration tool 30 comprises a chassis 31 and a holding bracket 32, and the holding bracket 32 is set on the chassis 31 for holding the measured object 40 mounted with the tracking marker 21, such that the holding bracket 32 makes the direction of the straight line L for the measured object 40 remain unchanged in the three-dimensional space, while allowing the measured object 40 to rotate along the axis of the set straight line L.

In some implementations for better accuracy of the measuring the directional calibration parameters, the line set for the measured object is disposed not parallel to any axis of X,Y,Z in the coordinates defined by the positional tracking apparatus. The angels between the line and the X, Y, Z axes are set to be acute angles, for example, between 30 degree to 60 degree.

In some embodiments, the method of determining the directional calibration parameters for the object comprises: keeping the linear direction unchanged for the object, obtaining plurality of rotating status parameters to deduce the directional calibration parameters with the formula of two-point position relationship or linear direction rotation relationship in the three-dimensional space. Various modifications, deductions of equations 10)- 12) and 1-1) - 1-12) are possible.

2. Determining the direction for a surgical instrument

Determining the directional calibration parameters for the surgical instrument may be performed and completed before actually surgical operation. The determined directional calibration parameters can be used in determining the spatial action direction of the surgical instrument during procedures, for example, the action direction for the surgical instrument to move around in a patient's body.

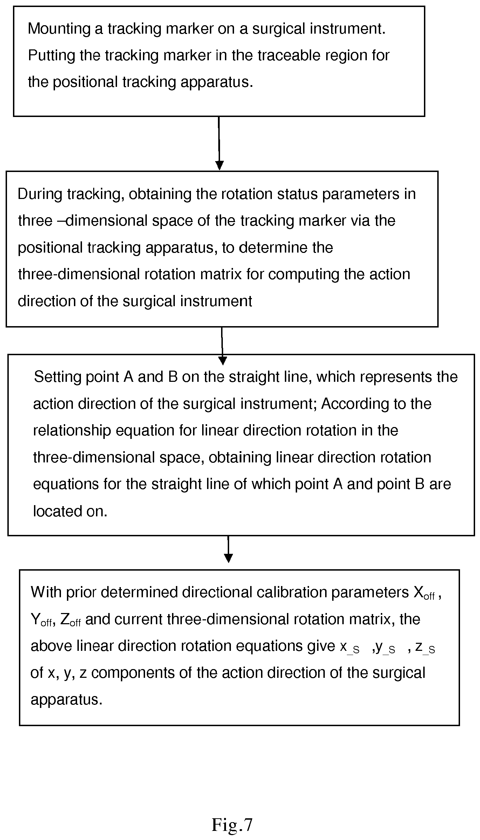

Figure 7 shows a flow chart of an embodiment of the method for determining the action direction for the surgical instrument. As shown in Figure 7, the method comprises the steps of:

Step 1) mounting a tracking marker on the surgical apparatus, in a way that the relative direction in the three-dimensional space between the direction of the straight line for the surgical apparatus and the tracking marker is the same as the relative direction in the three-dimensional space between the direction of the straight line for the measured object and the tracking marker during the determination of the directional calibration parameters, and putting the tracking marker in the traceable region for the position tracking apparatus;

Step 2) during tracking, obtaining the rotation status parameters of the tracking marker in the three dimensional space via the position tracking apparatus, to determine the three-dimensional rotation matrix for computing the action direction;

Step 3) setting point A and point B on the set straight line, which represents the action direction of a surgical apparatus; setting three-dimensional coordinates A $(XA\_s, Y_{A\_S}, Z_{A\_S})$ and B $(XB\_s, Y_{B\_S}, Z_{B\_S})$ respectively.

According to the formula of linear direction rotation relationship , equation r)-t) are obtained:

$$\delta x_{\_S} = X_{off} \times M_S(1,1) + Y_{off} \times M_S(2,1) + Z_{off} \times M_S(3,1) \qquad r)$$

$$\delta y_{\_S} = X_{off} \times M_S(1,2) + Y_{off} \times M_S(2,2) + Z_{off} \times M_S(3,2) \qquad s) \ ;$$

$$\delta z_{\_S} = X_{off} \times M_S(1,3) + Y_{off} \times M_S(2,3) + Z_{off} \times M_S(3,3) \qquad \tau) \ ;$$

Wherein: $X_{off}$, $Y_{off}$, $Z_{off}$ are x, y, z components of directional calibration parameters for the set straight line for the surgical apparatus. $\delta_{X\_S} = X_{A\_S} - X_{B\_S}$, $\delta_{Y\_S} = Y_{A\_S} - Y_{B\_S}$, $\delta_{Z\_S} = Z_{A\_S} - Z_{B\_S}$, wherein $\delta_{X\_S}$, $\delta_{Y\_S}$, $\delta_{Z\_S}$ represent X, Y, Z components of the linear direction in three-dimensional coordinate system. The matrix $M_s$

$$\begin{bmatrix} M_S(1,1) & M_S(1,2) & M_S(1,3) \\ M_S(2,1) & M_S(2,2) & M_S(2,3) \\ M_S(3,1) & M_S(3,2) & M_S(3,3) \end{bmatrix}$$

is the current three-dimensional rotation matrix for computing action direction determined from step 2).

Step 4)

With directional calibration parameters $X_{off}$, $Y_{off}$, $Z_{off}$ determined already and current three-dimensional rotation matrix $M_s$ determined from step 2), equations r)-t) just give $\delta_{X\_S}$, $\delta_{Y\_S}$, $\delta_{Z\_S}$ of x, y, z components of the action direction of the surgical apparatus.

It is noted for the formula of linear direction rotation relationship that when the tracking marker mounted on the surgical instrument is at "zero" direction, its three rotation angles will be all "zero", and its rotation matrix will be unit matrix. x, y, and z components of direction of line L can be expressed as $X_{off} = \delta x^{(0)}$, $Y_{off} = \delta y^{(0)}$, $Z_{off} = \delta z^{(0)}$. When the tracking marker is at any direction, its rotation matrix $M_s$ will not be unit matrix. The direction of line will be its "zero" direction $X_{off}$, $Y_{off}$, $Z_{off}$ (x, y, z components) multiplied by the rotation matrix, which is just what the equations r)-t) describe.

In some embodiments, the current three-dimensional rotation matrix Ms determined from step 2) for computing the action direction of the surgical instrument may be obtained from different rotation status parameters of the tracking marker. The rotation status parameters may be three rotation angles, three-dimensional rotation matrix provided by the positional tracking apparatus.

In some implementations, the tracking marker is disposed in front of the positional tracking apparatus, so that the tracking marker is in the traceable region and can be tracked to obtain its three-dimensional rotation status parameters, and there fore its three-dimensional rotation matrix.

In some implementations, the straight line set for the surgical instrument may be located on the surgical instrument, may also be located outside the surgical instrument, as long as the line represents the action direction of the surgical instrument.

The mounting of the tracking marker on the surgical instrument is fixed so that there is no relative direction change between the tracking marker and the surgical instrument in the three-dimensional space during tracking and determining the action direction of the surgical instrument.

The tracking marker fixed on the surgical instrument may be detachable. When reattaching the tracking marker, it is ensured that no relative direction change between the tracking marker and the surgical instrument in the three-dimensional space.

It may be noted that determining the action direction of the surgical instrument is not dependent on the positions of the two points A, B on the line. The determination of the action direction will not be affected, as long as the relative direction between the direction of the straight line for the surgical apparatus and the tracking marker is the same as the relative direction between the direction of the straight line for the measured object and the tracking marker during the determination of the directional calibration parameters,

The action direction of the surgical instrument is the direction that surgical instrument performs the procedure's direction, or the surgical instrument's function implements on the direction.

The three-dimensional rotation matrix Ms

$$\begin{bmatrix} M_S(1,1) & M_S(1,2) & M_S(1,3) \\ M_S(2,1) & M_S(2,2) & M_S(2,3) \\ M_S(3,1) & M_S(3,2) & M_S(3,3) \end{bmatrix}$$

is either obtained directly from the positional tracking apparatus, or derived from the rotation status parameters, which are provided by the positional tracking apparatus. 3.A tool used in determining the directional calibration parameters.

The present invention discloses a tool for determining the directional calibration parameters. As shown in Figure 3, the calibration tool 30 includes a chassis 31. A holding bracket 32 is set on the chassis 31 for holding the tracked object 40, which has a tracking marker 21 mounted on it. The holding bracket 32 ensures the direction of the set straight line L for the measured object 40 remain unchanged in the three-dimensional space, while the measured object 40 rotates along the axis of the set straight line L.

As shown in Figure 3, the bracket 32 may be provided in pairs. Each bracket 32 is provided with holding portion 33, which may be V-shaped, ring-shaped, arcshaped, or other shapes. At least two points at holding portion 33 will support the measured object 40, so that the measured object 40 may be stabilized at a fixed spatial direction, and can rotate along the set line L as it axis, while keeping the direction of L unchanged.

The size of the open degree of V-shape, or bend degree of arc/ring for the portion 33 can be adjusted. The distance between brackets 32 is adjustable. In some implementations, the calibration tool 30 is made of non-metal materials.

In some implementations, the rotation status is expressed in the form of Euler angles: azimuth angle A (Azimuth), elevation angle E (Elevation), and roll angle R (Roll). The three-dimensional rotation matrix M

$$\begin{bmatrix} M(1,1) & M(1,2) & M(1,3) \\ M(2,1) & M(2,2) & M(2,3) \\ M(3,1) & M(3,2) & M(3,3) \end{bmatrix}$$

has a relationship with the angle A, the angle E and the angle R in the form below:

$$M(1,1) = COS(E) \times COS(A)$$

$$M(1,2) = COS(E) \times SIN(A)$$

$$M(1,3) = -SIN(E)$$

$$M(2,1) = -(COS(R) \times SIN(A)) + (SIN(R) \times SIN(E) \times COS(A))$$

$$M(2,2) = (COS(R) \times COS(A)) + (SIN(R) \times SIN(E) \times SIN(A))$$

$$M(2,3)= SIN(R) \times COS(E)$$

$$M(3,1)= (SIN(R) \times SIN(A)) + (COS(R) \times SIN(E) \times COS(A))$$

$$M(3,2)= -(SIN(R) \times COS(A)) + (COS(R) \times SIN(E) \times SIN(A))$$

$$M(3,3)= COS(R) \times COS(E)$$

As such, each element of the matrix M can be determined.

When the angle A=0, the angle E=0 and the angle R=0, the three-dimensional rotation matrix M will become unit matrix.

$$\begin{bmatrix} 1 & 0 & 0 \\ 0 & 1 & 0 \\ 0 & 0 & 1 \end{bmatrix}$$

When the definitions for the positive/negative x, y, z axes are different, or the definitions for the positive/negative rotation angles are different, or the forms of angles are different, the elements in the three-dimensional rotation matrix M will be different. There may be different forms of rotation status other than Euler angles and there are different relationships with the three-dimensional rotation matrix. Regardless of those differences, the determining of directional calibration parameters will not be affected and there fore the determining of action direction will not be affected.

It is noted that the coordinates used in the tracking marker's positions, rotation angles, three-dimensional rotation matrices, in the measured object's displacing and in the surgical instrument's disposing are the coordinates of the positional tracking apparatus in the three-dimensional space. All parameters and computations described herein are with the coordinates of the positional tracking apparatus. The positional tracking apparatus may be used in a surgical navigation system. The positional tracking apparatus and tracking marker are public known apparatus. The obtaining of rotation angles, three-dimensional rotation matrix of tracking markers by the positional tracking apparatus is public a known technique.

Advantages

Compared with existing methods which need to place the surgical instrument in a known direction, the present invention's method of determining the directional calibration parameters does not have such a requirement. By applying the positional tracking apparatus, the directional calibration parameters can be computed via the rotation status parameters of the tracking marker mounted on the measured object (the surgical instrument or the replacement). The operation is easy.

Furthermore, the method of determining the directional calibration parameters does not require computing the calibration parameters for two-point set on the straight line representing the direction of the measured object, and has nothing to do with the positions of the two points.

The method for determining the direction for the surgical instrument is: obtaining current rotating status parameters, using prior known directional calibration parameters to deduce the direction. The method does not require determining a set of two points on the linear direction for tracked surgical instrument. The linear direction represents the action direction of the surgical instrument. The action direction information for some surgical instruments is important for doctors to achieve a precise procedure, a better surgical outcome.

The present invention is applicable to any surgical instrument that needs to determine some linear direction on whole or part of the instrument, such as surgical needles, surgical drills, and implant screws. One more example is a surgical clamp with flexible endoscope. Though its hose part has no linear direction, head for removal and treatment, has action direction. Its action direction can be determined by the present invention's methods. Further, the present invention is also applicable to the case that the linear direction is not from the surgical instrument, but is emitted from the surgical instrument. For example, radiation, lasers, or particles emitted from the surgical instrument have linear direction, which can also be determined by the present invention.

The present invention is applicable to any case that needs to determine the linear direction of an object with positional tracking apparatus. The action direction may be a linear direction on the whole tracked object, or on the part of the tracked object, or may be located outside.

The present invention's tool used specially for determining the directional calibration parameters plays a role to support, clamp and fix the measured object. The tool can assist the measured object to rotate along the set line as axis while keeping the direction of line unchanged. The tool can ensure fast and accurate measurement of the directional calibration parameters.

Although the invention is illustrated and described herein with reference to the preferred embodiments, the invention is not intended to be limited to the details shown. Rather, various modifications, such as deductions of equations, may be made in the details within the scope of the claims and without departing from the invention.

**Claims**

1. A method of determining directional calibration parameters of a surgical apparatus, the method comprising:

   a) disposing a tracking marker over a measured object, disposing the measured object on a calibration tool, and placing the tracking marker in a traceable region for a positional tracking apparatus;

   b) with the aid of the calibration tool, rotating the measured object around a set axis of a straight line, while keeping a direction of the straight line unchanged in a three-dimensional space;

   c) during rotation of the object, obtaining at least two groups of rotation status parameters of the tracking marker in at least two different rotation positions via the positional tracking apparatus, and from the at least two groups of rotation status parameters, determining at least two three-dimensional rotation matrices of the tracking marker for computing directional calibration parameters;

   **characterized in that** computing the directional calibration parameters comprises the steps of:

   d) setting arbitrary point A and arbitrary point B on the set straight line; setting three-dimensional coordinates A $(X_A, Y_A, Z_A)$ and B $(X_B, Y_B, Z_B)$ respectively;

   e) setting $\delta_X = X_A - X_B$, $\delta_Y = Y_A - Y_B$, $\delta_Z = Z_A - Z_B$, wherein $\delta_X$, $\delta_Y$, $\delta_Z$ represent x, y, z components of the set straight line in three-dimensional coordinate system;

   according to the formula of linear direction rotation relationship, equations 1 to 3 are obtained:

$$\delta_X = X_{off} \times M_P(1,1) + Y_{off} \times M_P(2,1) + Z_{off} \times M_P(3,1) \qquad 1);$$

$$\delta_Y = X_{off} \times M_P(1,2) + Y_{off} \times M_P(2,2) + Z_{off} \times M_P(3,2) \qquad 2);$$

$$\delta_Z = X_{off} \times M_P(1,3) + Y_{off} \times M_P(2,3) + Z_{off} \times M_P(3,3) \qquad 3);$$

   wherein: $X_{off}$, $Y_{off}$, $Z_{off}$ are x, y, z components of directional calibration parameters for the set straight line for the measured object;
   the matrix $M_P$

$$\begin{bmatrix} M_P(1,1) & M_P(1,2) & M_P(1,3) \\ M_P(2,1) & M_P(2,2) & M_P(2,3) \\ M_P(3,1) & M_P(3,2) & M_P(3,3) \end{bmatrix}$$

   is the $P^{th}$ matrix of three-dimensional rotation for computing directional calibration parameters determined from step c), p = 1, 2, ..., n, where n is a positive integer greater than 1; and $\delta_X$, $\delta_Y$, $\delta_Z$ and $X_{off}$, $Y_{off}$, $Z_{off}$ being unknowns in the inhomogeneous system of equations 1 to 3;

   f) substituting each three dimensional rotation matrix for computing directional calibration parameters determined from step c) into equations 1 to 3, using the formula for determining the distance between the two points, or having one of the components of the direction be a non-zero constant, to compute the directional calibration parameters $X_{off}$, $Y_{off}$, $Z_{off}$ by solving the inhomogeneous linear equations 1 to 3.

2. The method of claim 1, wherein determining the directional calibration parameters includes:

in step f) of claim 1, wherein using the formula for determining the distance between the two points is as follows:

the formula of distance D between point A and point B is:

$$D = \sqrt{\delta_X{}^2 + \delta_Y{}^2 + \delta_Z{}^2} \ ;$$

wherein: D is a known non-zero number constant; substituting each three-dimensional rotation matrix for computing directional calibration parameters determined from step c) of claim 1 into equations 1 to 3, plus the equation of D, constitutes simultaneous equations, solving the combined simultaneous equations gives $X_{off}$, $Y_{off}$, $Z_{off}$;
in step f) of claim 1, wherein the having one of the components of the direction be a non-zero constant is as follows:
among $\delta_X$, $\delta_Y$, $\delta_Z$ setting a non-zero component as known non-zero constant; substituting each three-dimensional rotation matrix for computing directional calibration parameters determined from step c) of claim 1 into equations 1 to 3, constitutes non-homogeneous linear equations, solving the non-homogeneous linear equations gives $X_{off}$, $Y_{off}$, $Z_{off}$.

3. A method of determining the action direction of a surgical apparatus for which directional calibration parameters have been determined according to the method of claim 1 or claim 2, the method comprising:

Step 1) mounting a tracking marker on the surgical apparatus, in a way that the relative direction or angle in the three-dimensional space between the direction of the straight line for the surgical apparatus and the tracking marker is the same as the relative direction or angle in the three dimensional space between the direction of the straight line for the measured object and the tracking marker during the determination of the directional calibration parameters, and putting the tracking marker in the traceable region for a position tracking apparatus;
Step 2) during tracking, obtaining the rotation status parameters of the tracking marker in the three-dimensional space via the position tracking apparatus, to determine the three-dimensional rotation matrix for computing the action direction;
Step 3) setting point A and point B on a straight line, which represents the action direction of a surgical apparatus; setting three-dimensional coordinates A ($X_{A\_S}$, $Y_{A\_S}$, $Z_{A\_S}$) and B ($X_{B\_S}$, $Y_{B\_S}$, $Z_{B\_S}$) respectively;

according to the formula of linear direction rotation relationship, equations r to t are obtained:

$$\delta_{X\_S} = X_{off} x M_S(1,1) + Y_{off} x M_S(2,1) + Z_{off} x M_S(3,1) \qquad r);$$

$$\delta_{Y\_S} = X_{off} x M_S(1,2) + Y_{off} x M_S(2,2) + Z_{off} x M_S(3,2) \qquad s);$$

$$\delta_{Z\_S} = X_{off} x M_S(1,3) + Y_{off} x M_S(2,3) + Z_{off} x M_S(3,3) \qquad t);$$

wherein: $X_{off}$, $Y_{off}$, $Z_{off}$ are x, y, z components of directional calibration parameters for the set straight line for the surgical apparatus; $\delta_{X\_S} = X_{A\_S} - X_{B\_S}$, $\delta_{Y\_S} = Y_{A\_S} - Y_{B\_S}$, $\delta_{Z\_S} = Z_{A\_S} - Z_{B\_S}$, where $\delta_{X\_S}$, $\delta_{Y\_S}$, $\delta_{Z\_S}$ represent x, y, z components of the action direction in the three-dimensional coordinate system; the matrix $M_s$

$$\begin{bmatrix} M_S(1,1) & M_S(1,2) & M_S(1,3) \\ M_S(2,1) & M_S(2,2) & M_S(2,3) \\ M_S(3,1) & M_S(3,2) & M_S(3,3) \end{bmatrix}$$

is the current three-dimensional rotation matrix for computing action direction determined from step 2);

Step 4) with directional calibration parameters $X_{off}$, $Y_{off}$, $Z_{off}$ determined from claim 1 or claim 2, and current three-dimensional rotation matrix $M_s$ determined from step 2), equations r to t just give $\delta_{X\_S}$, $\delta_{Y\_S}$, $\delta_{Z\_S}$ of x, y, z components of the action direction of the surgical apparatus.

4. The method of claim 3, wherein the action direction corresponds to a linear direction on the surgical apparatus, or on a part of the surgical apparatus, or located outside of the surgical apparatus.

5. The method of claim 3 or claim 4, wherein the action direction corresponds to a radiation, laser or particle emission direction from the surgical apparatus.

6. The method of any preceding claim, wherein the surgical apparatus is one of a surgical needle, surgical drill, implant screw or a surgical clamp with a flexible endoscope.

7. The method of any preceding claim, wherein the straight line is located on the surgical apparatus (40).

8. The method of any preceding claim, wherein the straight line is located outside of the surgical apparatus (40), and wherein the measured object is rotatable along the straight line as its rotation axis.

9. The method of any preceding claim, wherein the tracking marker is detachable from the surgical apparatus.

10. The method of any preceding claim, wherein the step of rotation of the object comprises manually or automatically rotating the object.


**Patentansprüche**

1. Verfahren zum Bestimmen gerichteter Kalibrationsparameter eines chirurgischen Geräts, wobei das Verfahren umfasst:

   a) Anordnen eines Verfolgungsmarkers über einem gemessenen Objekt, Anordnen des gemessenen Objekts auf einem Kalibrationswerkzeug, und Platzieren des Verfolgungsmarkers in einer verfolgbaren Region für ein Positionsverfolgungsgerät;
   b) Rotieren des gemessenen Objekts mit Hilfe des Kalibrationswerkzeugs um eine festgelegte Achse einer geraden Linie, während eine Richtung der geraden Linie in einem dreidimensionalen Raum unverändert gelassen wird;
   c) Erhalten von mindestens zwei Gruppen von Rotationsstatusparametern des Verfolgungsmarkers während der Rotation des Objekts in mindestens zwei verschiedenen Rotationspositionen mittels des Positionsverfolgungsgeräts, und Bestimmen von mindestens zwei dreidimensionalen Rotationsmatrizes des Verfolgungsmarkers aus den mindestens zwei Gruppen von Rotationsstatusparametern, um gerichtete Kalibrationsparameter zu berechnen;

   **dadurch gekennzeichnet, dass** Berechnen der gerichteten Kalibrationsparameter die Schritte umfasst:

   d) Festlegen des willkürlichen Punktes A und des willkürlichen Punktes B auf der festgelegten geraden Linie; Festlegen der jeweiligen dreidimensionalen Koordinaten A ($X_A$, $Y_A$, $Z_A$) und B ($X_B$, $Y_B$, $Z_B$) ;
   e) Festlegen von $\delta_X = X_A - X_B$, $\delta_Y = Y_A - Y_B$, $\delta_Z = Z_A - Z_B$, wobei $\delta_X$, $\delta_Y$, $\delta_Z$ für die Komponenten x, y, z der festgelegten geraden Linie in dem dreidimensionalen Koordinatensystem stehen;

   wobei gemäß der Formel der linearen gerichteten Rotationsbeziehungen die Gleichungen 1 bis 3 erhalten werden:

$$\delta_X = X_{off} x M_P(1,1) + Y_{off} x M_P(2,1) + Z_{off} x M_P(3,1) \qquad 1);$$

$$\delta_Y = X_{off} x M_P(1,2) + Y_{off} x M_P(2,2) + Z_{off} x M_P(3,2) \qquad 2);$$

$$\delta_Z = X_{off} x M_P(1,3) + Y_{off} x M_P(2,3) + Z_{off} x M_P(3,3) \qquad 3);$$

wobei: $X_{off}$, $Y_{off}$, $Z_{off}$ die Komponenten x, y, z der gerichteten Kalibrationsparameter für die festgelegte gerade Linie für das gemessene Objekt sind; die Matrix $M_P$

$$\begin{bmatrix} M_P(1,1) & M_P(1,2) & M_P(1,3) \\ M_P(2,1) & M_P(2,2) & M_P(2,3) \\ M_P(3,1) & M_P(3,2) & M_P(3,3) \end{bmatrix}$$

die P. Matrix der dreidimensionalen Rotation zum Berechnen der gerichteten Kalibrationsparameter ist, die aus Schritt c) bestimmt wurde, p = 1, 2,..., n, wobei n eine positive ganze Zahl größer als 1 ist; und $\delta_X$, $\delta_Y$, $\delta_Z$ und $X_{off}$, $Y_{off}$, $Z_{off}$ Unbekannte in dem inhomogenen System der Gleichungen 1 bis 3 sind;

f) Einsetzen von jeder dreidimensionalen Rotationsmatrix zum Berechnen gerichteter Kalibrationsparameter, die aus Schritt c) bestimmt wurden, in die Gleichungen 1 bis 3 unter Verwendung der Formel zur Bestimmung des Abstands zwischen zwei Punkten, oder wobei eine der Komponenten der Richtung eine Konstante ungleich Null ist, um die gerichteten Kalibrationsparameter $X_{off}$, $Y_{off}$, $Z_{off}$ zu berechnen, indem die inhomogenen linearen Gleichungen 1 bis 3 gelöst werden.

2. Verfahren nach Anspruch 1, wobei Bestimmen der gerichteten Kalibrationsparameter einschließt:

in Schritt f) von Anspruch 1, wobei die Verwendung der Formel zum Bestimmen des Abstands zwischen den beiden Punkten wie folgt ist:

ist die Formel des Abstands D zwischen Punkt A und Punkt B:

$$D = \sqrt{\delta_X{}^2 + \delta_Y{}^2 + \delta_Z{}^2} \; ;$$

wobei: D eine bekannte Zahlenkonstante ungleich Null ist; Einsetzen jeder dreidimensionalen Rotationsmatrix zum Berechnen der gerichteten Kalibrationsparameter, die aus Schritt c) von Anspruch 1 bestimmt wurden, in die Gleichungen 1 bis 3, plus die Gleichung D, wodurch simultane Gleichungen gestellt werden, wobei Lösen der kombinierten simultanen Gleichungen $X_{off}$, $Y_{off}$, $Z_{off}$ ergibt;
in Schritt f) von Anspruch 1, wobei das Vorliegen von einer der Komponenten der Richtung als Konstante ungleich Null wie folgt ist:
innerhalb von $\delta_X$, $\delta_Y$, $\delta_Z$ Festlegen einer Komponente ungleich Null als bekannte Konstante ungleich Null; Einsetzen von jeder dreidimensionalen Rotationsmatrix zum Berechnen von gerichteten Kalibrationsparametern, die aus Schritt c) von Anspruch 1 bestimmt wurde, in Gleichungen 1 bis 3, wodurch nicht-homogene lineare Gleichungen gebildet werden, wobei Lösen der nicht-homogenen linearen Gleichungen $X_{off}$, $Y_{off}$, $Z_{off}$ ergibt.

3. Verfahren zum Bestimmen der Aktionsrichtung eines chirurgischen Geräts, für das gerichtete Kalibrationsparameter gemäß dem Verfahren von Anspruch 1 oder Anspruch 2 bestimmt worden sind, wobei das Verfahren umfasst:

Schritt 1) Montieren eines Verfolgungsmarkers an das chirurgische Gerät in einer Weise, dass die relative Richtung oder der relative Winkel in dem dreidimensionalen Raum zwischen der Richtung der geraden Linie für das chirurgische Gerät und den Verfolgungsmarker die gleiche wie die relative Richtung oder der relative Winkel in dem dreidimensionalen Raum zwischen der Richtung der geraden Linie für das gemessene Objekt und den Verfolgungsmarker während der Bestimmung der gerichteten Kalibrationsparameter ist, und Setzen des Verfolgungsmarkers in eine verfolgbare Region für ein Positionsverfolgungsgerät;
Schritt 2) Erhalten der Rotationsstatusparameter des Verfolgungsmarkers im dreidimensionalen Raum während des Verfolgens mittels des Positionsverfolgungsgeräts, um die dreidimensionale Rotationsmatrix zur Berechung der Aktionsrichtung zu bestimmen;
Schritt 3) Festlegen von Punkt A und Punkt B auf einer geraden Linie, welche die Aktionsrichtung eines chirurgischen Geräts repräsentiert; Festlegen der dreidimensionalen Koordinaten A $(X_{A\_S}, Y_{A\_S}, Z_{A\_S})$ beziehungsweise B $(X_{B\_S}, Y_{B\_S}, Z_{B\_S})$;

wobei gemäß der Formel der linearen gerichteten Rotationsbeziehungen die Gleichungen r bis t erhalten werden:

$$\delta_{X\_S} = X_{off} \times M_S(1,1) + Y_{off} \times M_S(2,1) + Z_{off} \times M_S(3,1) \quad r);$$

$$\delta_{Y\_S} = X_{off} \times M_S(1,2) + Y_{off} \times M_S(2,2) + Z_{off} \times M_S(3,2) \quad s);$$

$$\delta_{Z\_S} = X_{off} \times M_S(1,3) + Y_{off} \times M_S(2,3) + Z_{off} \times M_S(3,3) \quad t);$$

wobei: $X_{off}$, $Y_{off}$, $Z_{off}$ die Komponenten x, y, z der gerichteten Kalibrationsparameter für die festgelegte gerade Linie für das chirurgische Gerät sind; $\delta_{X\_S} = X_{A\_S}-X_{B\_S}$, $\delta_{Y\_S} = Y_{A\_S}-Y_{B\_S}$, $\delta_{Z\_S} = Z_{A\_S}-Z_{B\_S}$, wobei $\delta_{X\_S}$, $\delta_{Y\_S}$, $\delta_{Z\_S}$ die Komponenten x, y, z in der Aktionsrichtung in dem dreidimensionalen Koordinatensystem repräsentieren; die Matrix $M_s$

$$\begin{bmatrix} M_S(1,1) & M_S(1,2) & M_S(1,3) \\ M_S(2,1) & M_S(2,2) & M_S(2,3) \\ M_S(3,1) & M_S(3,2) & M_S(3,3) \end{bmatrix}$$

die aktuelle dreidimensionale Rotationsmatrix zum Berechnen der Aktionsrichtung ist, die aus Schritt 2) bestimmt wurde;

Schritt 4) wobei die gerichteten Kalibrationsparameter $X_{off}$, $Y_{off}$, $Z_{off}$, die aus Anspruch 1 und Anspruch 2 bestimmt wurden, und die aktuelle dreidimensionale Rotationsmatrix $M_s$, die aus Schritt 2) bestimmt wurde, Gleichungen r bis t direkt $\delta_{X\_S}$, $\delta_{Y\_S}$, $\delta_{Z\_S}$ der Komponenten x, y, z der Aktionsrichtung des chirurgischen Geräts ergeben.

4. Verfahren nach Anspruch 3, wobei die Aktionsrichtung einer linearen Richtung auf dem chirurgischen Gerät oder auf einem Teil des chirurgischen Geräts entspricht oder sich außerhalb des chirurgischen Geräts befindet.

5. Verfahren nach Anspruch 3 oder Anspruch 4, wobei die Aktionsrichtung einer Strahlungs-, Laser- oder Partikelemissionsrichtung von dem chirurgischen Gerät entspricht.

6. Verfahren nach einem der vorhergehenden Ansprüche, wobei das chirurgische Gerät eines von einer chirurgischen Nadel, einem chirurgischen Bohrer, einer Implantatschraube oder chirurgischen Klemme mit einem flexiblen Endoskop ist.

7. Verfahren nach einem der vorhergehenden Ansprüche, wobei die gerade Linie sich auf dem chirurgischen Gerät (40) befindet.

8. Verfahren nach einem der vorhergehenden Ansprüche, wobei die gerade Linie sich außerhalb des chirurgischen Geräts (40) befindet, und wobei das gemessene Objekt entlang der geraden Linie als seiner Rotationsachse rotierbar ist.

9. Verfahren nach einem der vorhergehenden Ansprüche, wobei der Verfolgungsmarker von dem chirurgischen Gerät abnehmbar ist.

10. Verfahren nach einem der vorhergehenden Ansprüche, wobei der Schritt der Rotation des Objekts manuelles oder automatisches Rotieren des Objekts umfasst.

**Revendications**

1. Procédé de détermination de paramètres d'étalonnage directionnel d'un appareil chirurgical, le procédé comprenant :

a) la disposition d'un marqueur de suivi sur un objet mesuré, la disposition de l'objet mesuré sur un outil d'étalonnage, et le placement du marqueur de suivi dans une région traçable pour un appareil de suivi positionnel ;

b) à l'aide de l'outil d'étalonnage, le fait de faire tourner l'objet mesuré autour d'un axe défini d'une ligne droite, tout en maintenant une direction de la ligne droite inchangée dans un espace tridimensionnel ;

c) pendant la rotation de l'objet, l'obtention d'au moins deux groupes de paramètres d'état de rotation du marqueur de suivi dans au moins deux positions de rotation différentes par l'intermédiaire de l'appareil de suivi de position, et à partir des au moins deux groupes de paramètres d'état de rotation, la détermination d'au moins deux matrices de rotation tridimensionnelles du marqueur de suivi pour calculer les paramètres de calibrage directionnel ;

**caractérisé en ce que** le calcul des paramètres de calibrage directionnel comprend les étapes de :

d) définition d'un point arbitraire A et d'un point arbitraire B sur la ligne droite définie ; définition des coordonnées tridimensionnelles A $(X_A, Y_A, Z_A)$ et B $(X_B, Y_B, Z_B)$ respectivement ;

e) définition de $\delta_X = X_A - X_B$, $\delta_Y = Y_A - Y_B$, $\delta_Z = Z_A - Z_B$, avec $\delta_X$, $\delta_Y$, $\delta_Z$ représentant les composantes x, y, z de la ligne droite établie dans le système de coordonnées tridimensionnelles ;

selon la formule de la relation de rotation de la direction linéaire, on obtient les équations 1 à 3 :

$$\delta_X = X_{off} \times M_p(1,1) + Y_{off} \times M_p(2,1) + Z_{off} \times M_p(3,1) \qquad 1) \ ;$$

$$\delta_Y = X_{off} \times M_p(1,2) + Y_{off} \times M_p(2,2) + Z_{off} \times M_p(3,2) \qquad 2) \ ;$$

$$\delta_Z = X_{off} \times M_p(1,3) + Y_{off} \times M_p(2,3) + Z_{off} \times M_p(3,3) \qquad 3) \ ;$$

avec : $X_{off}$, $Y_{off}$, $Z_{off}$ étant des composantes x, y, z de paramètres d'étalonnage directionnel pour la ligne droite définie pour l'objet mesuré ;

la matrice $M_p$

$$\begin{bmatrix} M_P(1,1) & M_P(1,2) & M_P(1,3) \\ M_P(2,1) & M_P(2,2) & M_P(2,3) \\ M_P(3,1) & M_P(3,2) & M_P(3,3) \end{bmatrix}$$

étant la $P^{ième}$ matrice de rotation tridimensionnelle pour le calcul des paramètres d'étalonnage directionnel déterminés à l'étape c), p = 1,2,...,n, n étant un entier positif supérieur à 1 ; et $\delta_X$, $\delta_Y$, $\delta_Z$ et $X_{off}$, $Y_{off}$, $Z_{off}$ étant des inconnues dans le système inhomogène d'équations 1 à 3 ;

f) substitution de chaque matrice de rotation tridimensionnelle pour calculer les paramètres d'étalonnage directionnel déterminés à l'étape c) dans les équations 1 à 3, en utilisant la formule pour déterminer la distance entre les deux points, ou en faisant en sorte que l'une des composantes de la direction soit une constante non nulle, pour calculer les paramètres d'étalonnage directionnel $X_{off}$, $Y_{off}$, $Z_{off}$ en résolvant les équations linéaires inhomogènes 1 à 3.

**2.** Procédé selon la revendication 1, la détermination des paramètres de calibrage directionnel comprenant :

à l'étape f) selon la revendication 1, l'utilisation de la formule pour déterminer la distance entre les deux points telle que suit :

la formule de la distance D entre le point A et le point B est : $D = \sqrt{\delta_X^2 + \delta_Y^2 + \delta_Z^2}$ ;

D étant une constante numérique connue non nulle ; la substitution de chaque matrice de rotation tridimensionnelle pour le calcul des paramètres d'étalonnage directionnel déterminés à partir de l'étape c) selon la revendication 1 dans les équations 1 à 3, plus l'équation de D, constituant des équations simultanées, la résolution des équations simultanées combinées donnant $X_{off}$, $Y_{off}$, $Z_{off}$ ;

à l'étape f) selon la revendication 1, le fait que l'une des composantes de la direction soit une constante non nulle étant le suivant :

parmi $\delta_X$, $\delta_Y$, $\delta_Z$ la définition d'une composante non nulle comme constante non nulle connue ; la substitution de chaque matrice de rotation tridimensionnelle pour calculer les paramètres d'étalonnage directionnel déterminés à partir de l'étape c) selon la revendication 1 dans les équations 1 à 3, constitue des équations linéaires non homogènes, la résolution des équations linéaires non homogènes donne $X_{off}$, $Y_{off}$, $Z_{off}$.

3. Procédé de détermination de la direction d'action d'un appareil chirurgical pour lequel des paramètres de calibration directionnelle ont été déterminés par le procédé selon la revendication 1 ou selon la revendication 2, le procédé comprenant :

étape 1) le montage d'un marqueur de suivi sur l'appareil chirurgical, de manière à ce que la direction ou l'angle relatif dans l'espace tridimensionnel entre la direction de la ligne droite pour l'appareil chirurgical et le marqueur de suivi soit le même que la direction ou l'angle relatif dans l'espace tridimensionnel entre la direction de la ligne droite pour l'objet mesuré et le marqueur de suivi pendant la détermination des paramètres d'étalonnage directionnel, et le placement du marqueur de suivi dans la région traçable pour un appareil de suivi de position ;
étape 2) pendant le suivi, l'obtention des paramètres d'état de rotation du marqueur de suivi dans l'espace tridimensionnel par l'intermédiaire de l'appareil de suivi de position, afin de déterminer la matrice de rotation tridimensionnelle pour calculer la direction d'action ;
étape 3) la définition du point A et du point B sur une ligne droite, qui représente la direction d'action d'un appareil chirurgical ; la définition des coordonnées tridimensionnelles A ($X_{A\_S}$, $Y_{A\_S}$, $Z_{A\_S}$) et B ($X_{B\_S}$, $Y_{B\_S}$, $Z_{B\_S}$) respectivement ;
selon la formule de la relation de rotation de direction linéaire, on obtient les équations r à t :

$$\delta_{X\_S} = X_{off} \times M_S(1,1) + Y_{off} \times M_S(2,1) + Z_{off} \times M_S(3,1) \quad r) \quad ;$$

$$\delta_{Y\_S} = X_{off} \times M_S(1,2) + Y_{off} \times M_S(2,2) + Z_{off} \times M_S(3,2) \quad s) \quad ;$$

$$\delta_{Z\_S} = X_{off} \times M_S(1,3) + Y_{off} \times M_S(2,3) + Z_{off} \times M_S(3,3) \quad t) \quad ;$$

avec : $X_{off}$, $Y_{off}$, $Z_{off}$ étant des composantes x, y, z de paramètres d'étalonnage directionnel pour la ligne droite établie pour l'appareil chirurgical ; $\delta_{X\_S} = X_{A\_S} - X_{B\_S}$, $\delta_{Y\_S} = Y_{A\_S} - Y_{B\_S}$, $\delta_{Z\_S} = Z_{A\_S} - Z_{B\_S}$, avec $\delta_{X\_S}$, $\delta_{Y\_S}$, $\delta_{Z\_S}$ représentant des composantes x, y, z de la direction d'action dans le système de coordonnées tridimensionnelles ; la matrice Ms

$$\begin{bmatrix} M_S(1,1) & M_S(1,2) & M_S(1,3) \\ M_S(2,1) & M_S(2,2) & M_S(2,3) \\ M_S(3,1) & M_S(3,2) & M_S(3,3) \end{bmatrix}$$

étant la matrice de rotation tridimensionnelle actuelle pour le calcul de la direction de l'action déterminée à l'étape 2) ;
étape 4) avec les paramètres de calibrage directionnel $X_{off}$, $Y_{off}$, $Z_{off}$ déterminés selon la revendication 1 ou selon la revendication 2, et la matrice de rotation tridimensionnelle courante Ms déterminée à partir de l'étape 2), les équations r à t donnent juste $\delta_{X\_S}$, $\delta_{Y\_S}$, $\delta_{Z\_S}$ des composantes x, y, z de la direction d'action de l'appareil chirurgical.

4. Procédé selon la revendication 3, la direction d'action correspondant à une direction linéaire sur l'appareil chirurgical, ou sur une partie de l'appareil chirurgical, ou située à l'extérieur de l'appareil chirurgical.

5. Procédé selon la revendication 3 ou selon la revendication 4, la direction d'action correspondant à une direction d'émission de rayonnement, de laser ou de particules à partir de l'appareil chirurgical.

6. Procédé selon n'importe quelle revendication précédente, l'appareil chirurgical étant l'un parmi une aiguille chirurgicale, une perceuse chirurgicale, une vis d'implant ou une pince chirurgicale avec un endoscope flexible.

7. Procédé selon n'importe quelle revendication précédente, la ligne droite étant située sur l'appareil chirurgical (40).

8. Procédé selon n'importe quelle revendication précédente, la ligne droite étant située à l'extérieur de l'appareil chirurgical (40), et l'objet mesuré pouvant tourner le long de la ligne droite comme son axe de rotation.

9. Procédé selon n'importe quelle revendication précédente, le marqueur de suivi étant détachable de l'appareil chirurgical.

10. Procédé selon n'importe quelle revendication précédente, l'étape de rotation de l'objet comprenant la rotation manuelle ou automatique de l'objet.

11

10

**Fig.1**

21  40

L  41

A  O

B

30

**Fig. 2**

21  33

L  41

A  O  B  40

30

31  32

**Fig.3**

Mounting a tracking marker on a measured object, then placing the measured object on a calibration tool, and having the tracking marker in the traceable region for a positional tracking apparatus.

With the aid of the calibration tool, rotating the measured object along a set axis of a straight line, while keeping the direction of the straight line unchanged in three-dimensional space.

During rotation of the measured object, obtaining at least two groups of rotation status parameters of the tracking marker in at least two different rotation positions via the positional tracking apparatus. From the groups of rotation status parameters, determining at least two three-dimensional rotation matrices for computing the directional calibration parameters.

Setting point A and point B on the straight line apart distance D.

Substituting three-dimensional coordinates of A, B and representing point O of the tracking marker into the formula of two-point position relationship, obtaining relationship equations for A-O and relationship equations for B-O.

Substituting each three-dimensional rotation matrix for computing the directional calibration parameters into the above relationship equations, using the formula for determining the distance between the two points, or having one of the components of the direction be a non-zero constant, to compute the directional calibration parameters $X_{off}$, $Y_{off}$, $Z_{off}$

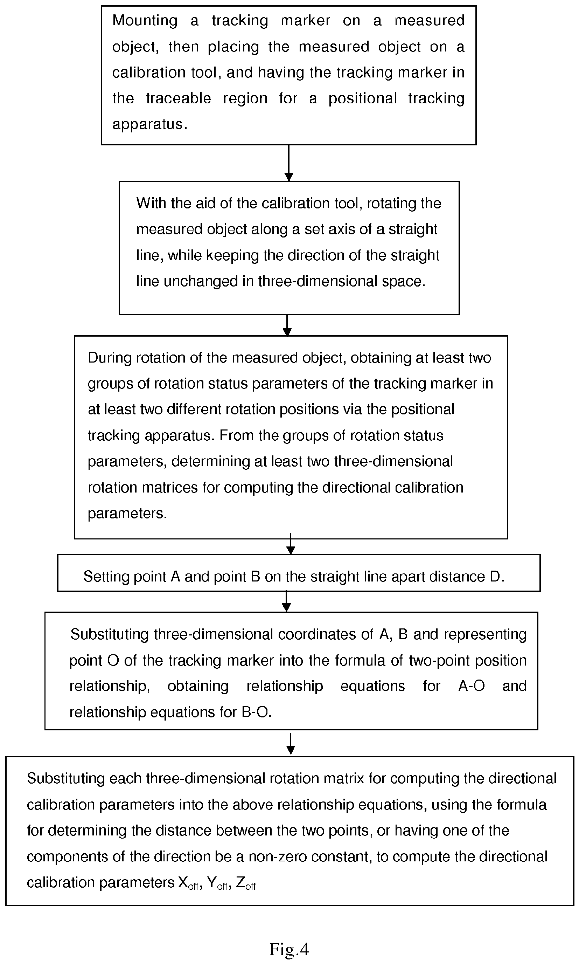

Fig.4

Mounting a tracking marker on a measured object, then placing the measured object on a calibration tool, and having the tracking marker in the traceable region for a positional tracking apparatus.

With the aid of the calibration tool, rotating the measured object along a set axis of a straight line, while keeping the direction of the straight line unchanged in three-dimensional space.

During rotation of the measured object, obtaining at least two groups of rotation status parameters of the tracking marker in at least two different rotation positions via the positional tracking apparatus. From the groups of rotation status parameters, determining at least two three-dimensional rotation matrices for computing the directional calibration parameters.

Setting point A and point B on the straight line apart distance D.

According to the relationship equation for linear direction rotation in the three-dimensional space, obtaining linear direction rotation equations for the straight line, where point A and point B are located on.

Substituting each three-dimensional rotation matrix for computing the directional calibration parameters into the above linear direction rotation equations, using the formula for determining the distance between the two points, or having one of the components of the direction be a non-zero constant, to compute the directional calibration parameters $X_{off}$, $Y_{off}$, $Z_{off}$ .

Fig.5

Mounting a tracking marker on a surgical instrument. Putting the tracking marker in the traceable region for the positional tracking apparatus.

During tracking, obtaining the rotation status parameters in three –dimensional space of the tracking marker via the positional tracking apparatus, to determine the three-dimensional rotation matrix for computing the action direction of the surgical instrument

Setting point A and B on the straight line, which represents the action direction of the surgical instrument; Setting point O to represent the tracking marker; Substituting three-dimensional coordinates of A, B and O into the formula of two-point position relationship, obtaining relationship equations for A-O and relationship equations for B-O.

Converting the relationship equations for A-O and relationship equations for B-O into linear direction rotation equations for $X_{off}$, $Y_{off}$, $Z_{off}$ and $\delta x_{\_S}$, $\delta y_{\_S}$, $\delta z_{\_S}$.

With prior determined directional calibration parameters $X_{off}$, $Y_{off}$, $Z_{off}$ and current three-dimensional rotation matrix, the above linear direction rotation equations give $x_{\_S}$, $y_{\_S}$, $z_{\_S}$ of x, y, z components of the action direction of the surgical apparatus.

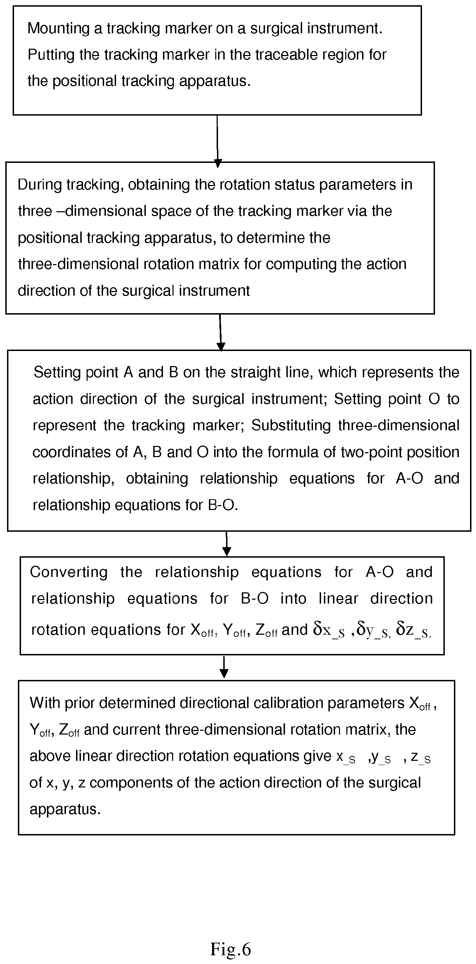

Fig.6

Mounting a tracking marker on a surgical instrument. Putting the tracking marker in the traceable region for the positional tracking apparatus.

During tracking, obtaining the rotation status parameters in three –dimensional space of the tracking marker via the positional tracking apparatus, to determine the three-dimensional rotation matrix for computing the action direction of the surgical instrument

Setting point A and B on the straight line, which represents the action direction of the surgical instrument; According to the relationship equation for linear direction rotation in the three-dimensional space, obtaining linear direction rotation equations for the straight line of which point A and point B are located on.

With prior determined directional calibration parameters $X_{off}$, $Y_{off}$, $Z_{off}$ and current three-dimensional rotation matrix, the above linear direction rotation equations give $x_s$, $y_s$, $z_s$ of x, y, z components of the action direction of the surgical apparatus.

Fig.7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2009247861 A1